(19) Europäisches Patentamt
European Patent Office
Office européen des brevets

(11) Publication number: **0 254 068 B1**

(12) **EUROPEAN PATENT SPECIFICATION**

(45) Date of publication of patent specification: **26.08.92**

(51) Int. Cl.$^5$: **A61K 31/445**

(21) Application number: **87109143.5**

(22) Date of filing: **25.06.87**

The file contains technical information submitted after the application was filed and not included in this specification

(54) **Novel therapeutic agent for gastritis.**

(30) Priority: **26.06.86 JP 150592/86**

(43) Date of publication of application:
**27.01.88 Bulletin 88/04**

(45) Publication of the grant of the patent:
**26.08.92 Bulletin 92/35**

(84) Designated Contracting States:
**BE CH DE FR GB IT LI NL SE**

(56) References cited:

**OYO YAKURI, vol. 17, no. 3, 1979, pages 371-382; T. IRIKURA et al.: "Preventive Effects of 3-(3,4,5-Trimethoxybenzamido) piperidine (KU-54) on Various Experimental Gastric or Duodenal Ulcers"**

**NIPPON YAKURIGAKU ZASSHI, vol. 89, no. 3, March 1987, pages 111-119; H. SEKIGUCHI et al.: "Effects of troxipide on acute gastric lesions in rats"**

(73) Proprietor: **KYORIN SEIYAKU KABUSHIKI KAISHA**
**2-5, Kanda Surugadai**
**Chiyoda-ku Tokyo(JP)**

(72) Inventor: **Kasuga, Kazunori**
**1-21-12, Harigaya**
**Urawa-shi Saitama-ken(JP)**
Inventor: **Sekiguchi, Haruo**
**1-5-4, Oaza Idoki**
**Ageo-shi Saitama-ken(JP)**
Inventor: **Hamada, Katsuhiro**
**5932, Tomonuma, Nogi-cho**
**Shimotsuga-gun Tochigi-ken(JP)**
Inventor: **Imai, Jun**
**1400-70, Kosugaya-cho, Sakae-ku**
**Yokohama-shi Kanagawa-ken(JP)**
Inventor: **Kamijo, Shinji**
**5-38-10, Soshigaya Setagaya-ku**
**Tokyo(JP)**

(74) Representative: **Vossius & Partner**
**Siebertstrasse 4 P.O. Box 86 07 67**
**W-8000 München 86(DE)**

**EP 0 254 068 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**OYO YAKURI, vol. 32, no. 2, 1986, pages 387-395; T. MOCHIZUKI et al.: "Effects of 3,4,5-Trimethoxy-N-3-piperidylbenzamide (Troxipide). an Anti-ulcer Anti-ulcer Agent, on Gastric Mucosal Prostaglandins"**

**"The Merck Manual of Diagnosis and Therapy", 15th Edition, 1987, pages 734-750**

**OYO YAKURI, vol. 15, no. 4, 1978, pages 641-651; T. IRIKURA et al.: "Curative Effects and Combination Effects with Other Drugs of 3-(3,4,5-Trimethoxybenzamido) Piperidine (KU-54) on Experimental Chronic Gastric Ulcers"**

**NIPPON YAKURIGAKU ZASSHI, vol. 83, no. 4, April 1984, pages 317-324; Y. ABE et al.: "Effects of 3,4,5-trimethoxy-N-(3-piperidyl) benzamide (KU-54) on the respiration of the gastric mucosa and liver in rats"**

## Description

Gastritis is pathologically an inflammatory process of the stomach, particularly of the gastric mucosa. It has been known that acute gastritis is often induced by ingestion of anti-inflammatory agents (aspirin,) or alcohol (ethanol), by emotional stress or by the flow-back of bile into the stomach. Furthermore, it may occasionally result from a mistaken ingestion of corrosive acid or alkali.

3,4,5-trimethoxy-N-3-piperidylbenzamide (international non-proprietary name (INN): troxipide) having the chemical structure shown below has been described by T. Irikura, K. Kasuga and M. Segawa as an anti-ulcer compound (Japanese Patent Publication No. Sho 50-28436). There are many reports on the anti-ulcer effects of troxipide both experimentally and clinically, while therapeutic effects of troxipide on gastritis have never been publicly known.

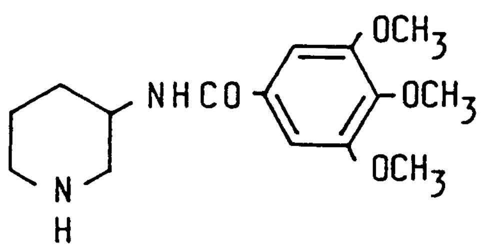

The object of the present invention is the provision of a novel therapeutic agent containing troxipide which is effective for the treatment of gastritis.

The present invention has been achieved by our discovery of prominent effects of troxipide on acute gastric lesions which are known as experimental models of gastritis. The present inventors have searched for an effective compound which protects gastric lesions induced by aspirin, 0.6 N HCl, water-immersion stress and ethanol, respectively, in rats, and it has been revealed that troxipide is highly effective though it differs entirely in chemical structure from the known effective compounds for gastritis.

As compared with cetraxate hydrochloride, an active ingredient of a clinically available agent for gastritis, troxipide shows much more potent protecting effects against gastric lesions induced by aspirin, 0.6 N HCl and water-immersion stress. Troxipide has almost equal potency to that of cetraxate hydrochloride against ethanol-induced gastric lesions. While it is known that troxipide can be used as an effective and safe therapeutic agent for the treatment of ulcers in humans, it has now been discovered that troxipide is effective for the treatment of gastritis in humans.

The pharmaceutical composition of the present invention may be administered orally, for example in the form of tablets, capsules, granules or fine granules. These formulations can be prepared by usual pharmaceutical techniques using a vehicle such as starch or low-substituted hydroxypropylcellulose as an excipient, hydroxypropyl-cellulose or carboxymethylcellulose as a binder, magnesium stearate or starch as a lubricant and colour, taste and flavour-providing compounds as additives.

The preferable amount of troxipide is 150-300 mg t.i.d. It can be adjusted according to the severity of the gastritis and/or to the age of the patients.

The following examples will further illustrate the present invention.

Example 1

Aspirin-Induced Gastric Lesions.

Rats weighing about 200 g were fasted for 48 hours and deprived of water for 24 hours. Thereafter, aspirin (Merck) at a dose level of 125 mg/kg was given orally 2 times every 2 hours. The animals were killed 3 hours after the second administration of aspirin. The stomach of each animal was removed and fixed in a 0.5 % neutral formalin solution according to the method of Brodie and Hanson (Gastroenterology 38, 353-360, 1960). The length (mm) of each of the gastric lesions was measured under a dissecting microscope (3 magnifications), summed, and used as an index for evaluation. Troxipide (100, 200 and 300 mg/kg) and cetraxate hydrochloride (100, 300 and 1,000 mg/kg) were orally given one hour before the first administration of aspirin, respectively.

Troxipide (200 and 300 mg/kg) significantly prevented the gastric lesions by 54.4 and 58.7 %, respectively, while cetraxate hydrochloride significantly prevented them by 37.7 % only at the dose level of 1,000 mg/kg.

Example 2

0.6 N HCl-Induced Gastric Lesions.

Rats weighing about 200 g were deprived of food and water for 24 hours. One ml of 0.6 N HCl solution was given orally and the animals were killed an hour later. Measurement of the gastric lesions was carried out according to the method described in Example 1. Troxipide (100, 200 and 300 mg/kg) and cetraxate hydrochloride (100, 300 and 1,000 mg/kg) were given orally one hour before the administration of 0.6 N HCl solution, respectively.

Troxipide (200 and 300 mg/kg) significantly prevented the gastric lesions by 48.6 and 55.6 %, respectively, while cetraxate hydrochloride significantly prevented them by 63.5 % only at the dose

level of 1,000 mg/kg.

Example 3

Water-Immersion Stress-Induced Gastric Lesions.

Rats weighing about 250 g were deprived of food but allowed free access to water for 24 hours. The animals were then immersed vertically to the level of the xiphoid provess in a water bath (23 °C) for 7 hours according to the method of Takagi and Okabe (The Japanese Journal of Pharmacology 18, 9-18, 1968) and then killed. Measurement of the gastric lesions was carried out according to the method described in Example 1. Troxipide (100, 200 and 300 mg/kg) and cetraxate hydrochloride (100, 300, 600 and 1,000 mg/kg) were given orally 10 minutes before subjecting the animals to water-immersion stress.

Troxipide (100, 200 and 300 mg/kg) significantly prevented the gastric lesions by 49.8, 73.7 and 84.9 %, respectively, whereas cetraxate hydrochloride (600 and 1,000 mg/kg) significantly prevented them by 53.2 and 52.0 %, respectively.

Example 4

Ethanol-Induced Gastric Lesions.

Rats weighing about 200 g were deprived of food and water for 24 hours. One ml of absolute ethanol was given orally and the animals were killed an hour later. Measurement of the gastric lesions was carried out according to the method described in Example 1. Troxipide (100, 200 and 300 mg/kg) and cetraxate hydrochloride (100, 300 and 1,000 mg/kg) were given orally one hour before the administration of ethanol, respectively.

Troxipide (100, 200 and 300 mg/kg) significantly prevented the gastric lesions by 50.7, 60.4 and 79.5 %, respectively. Cetraxate hydrochloride (100, 300 and 1,000 mg/kg) also significantly prevented them by 45.3, 67.8, 81.9%, respectively.

The pharmaceutical preparation of the present invention is illustrated by the following examples.

Example 5

500 g of troxipide was blended with 375 g of dried corn-starch and 200 g of low-substituted hydroxypropylcellulose. Purified water was then added, and the resultant mixture was kneaded by the usual method of mass granulation. 5 g of magnesium stearate was mixed with the granules to prepare tablets, each tablet having a weight of 108 mg (contained 50 mg of troxipide). The tablets thus prepared were coated with a coating material by the usual spraying method to yield film coated tablets having a weight of 110 mg each.

Example 6

1,000 g of troxipide was blended with 600 g of dried corn-starch and 350 g of low-substituted hydroxypropylcellulose. Purified water was then added, and the resultant mixture was kneaded by the usual method of mass granulation. 10 g of stearic acid was mixed with the granules to prepare tablets, each tablet having a weight of 196 mg (contained 100 mg of troxipide). The tablets thus prepared were coated with a coating material by the usual spraying method to yield film coated tablets having a weight of 200 mg each.

Example 7

2,400 g of troxipide was blended with 9,120 g of low-substituted hydroxypropylcellulose. This mixed powder was kneaded after adding an aqueous solution containing 360 g of dissolved hydroxypropylcellulose as a binder. The resulting mass was treated to granulate, using 0.5 mm screening, drying and sifting. The granules thus obtained were mixed with 120 g of dried cornstarch to yield fine granules containing 20 % of troxipide.

## Claims

1. The use of 3,4,5-trimethoxy-N-3-piperidylbenzamide (troxipide) for the preparation of a pharmaceutical composition for the prevention and treatment of gastritis.

## Patentansprüche

1. Verwendung von 3,4,5-Trimethoxy-N-3-piperidylbenzamid (Troxipide) zur Herstellung eines Arzneimittels zur Vorbeugung und Behandlung von Gastritis.

## Revendications

1. Utilisation de 3,4,5-triméthoxy-N-3-pipéridylbenzamide (troxipide) pour la préparation d'une composition pharmaceutique pour la prévention et le traitement de la gastrite.